# EUROPEAN PATENT APPLICATION

(11) **EP 3 263 022 A1**
(43) Date of publication of application: **03.01.2018**
(21) Application number: 17157340.5
(22) Date of filing: 22.02.2017
(51) Int. Cl.: A61B 5/0205, A61B 5/18, G08B 21/04, A61B 5/00, A61B 5/024, A61B 5/0476, A61B 5/08

(54) **ABNORMALITY PROCESSING SYSTEM**

(30) Priority: 30.06.2016 JP 2016129600
(71) Applicant: Omron Corporation, Kyoto 600-8530 (JP)
(72) Inventor: KOEZUKA, Yahiro, Kyoto-shi, Kyoto 600-8530 (JP); TAKAYAMA, Masahiro, Kyoto-shi, Kyoto 600-8530 (JP); KATO, Yutaro, Kyoto-shi, Kyoto 600-8530 (JP); MATSUI, Shigeru, Kyoto-shi, Kyoto 600-8530 (JP); KONISHI, Hidefumi, Kyoto-shi, Kyoto 600-8530 (JP); KASAI, Hirokazu, Kyoto-shi, Kyoto 600-8530 (JP); HIRAKI, Shigetsugu, Kyoto-shi, Kyoto 600-8530 (JP); MURAKI, Shiko, Kyoto-shi, Kyoto 600-8530 (JP); KAMEDA, Takamasa, Kyoto-shi, Kyoto 600-8530 (JP); OIKAWA, Takahiro, Kyoto-shi, Kyoto 600-8530 (JP)
(74) Representative: Becker Kurig Straus

(57) **Abstract**

An abnormality processing system is provided capable of executing appropriate processing in cases where an abnormality occurs in a worker's living body. A management computer 300, if determining that an abnormality is present in a worker's living body, transmits an abnormality signal to an administrator terminal 100, a work device 200, a worker camera 20 and a surveillance camera 50. When receiving the abnormality signal, the administrator terminal 100 displays an image showing that an abnormality has occurred. When receiving the abnormality signal, the work device 200 stops operation. When receiving the abnormality signal, the worker camera 20 and the surveillance camera 50 transmit to the management computer 300 imaging data containing at least data of images before and after a timing at which the abnormality has occurred in the worker's living body.

## Description

### BACKGROUND OF THE INVENTION

### Field of the Invention

The invention relates to an abnormality processing system executing predetermined abnormality processing.

### Description of the Related Art

Conventionally, at nursing and medical sites, vital sensors for detecting human vital signs such as body temperature and pulse have been used. For example, JP 2015-103116 discloses an automatic reporting system in which a vital sign of a patient in need of home care and of a patient who feels anxiety about their health is detected by a vital sensor, and when it is determined that an abnormality is present in the patient's living body based on a detected value of the vital sign, a medical institution will be so notified.

### [Prior-Art Documents]

### [Patent Documents]

Patent Document 1: JP 2015-103116

### SUMMARY OF THE INVENTION

### [Problems to Be Solved by the Invention]

At a work site such as a factory, although a manufacturing defect is likely to occur in cases where an abnormality is present in a worker's living body, it is difficult to prevent such occurrence of manufacturing defects if no measure is taken. In addition, since it is difficult to specify that the occurrence of the manufacturing defect is due to the abnormality in the worker's living body, it is also difficult to take a recurrence prevention measure. Like the automatic reporting system disclosed in Patent Document 1, at nursing and medical sites, since there is often anxiety about the patient's living body, appropriate processing is performed in cases where an abnormality occurs in the patent's living body. However, at a work site such as a factory, appropriate processing is not performed in cases where an abnormality occurs in a worker's living body.

The invention is made for solving the above problems, and provides an abnormality processing system capable of executing appropriate processing in cases where an abnormality occurs in a worker's living body.

### [Means for Solving the Problems]

According to an aspect of the invention, the abnormality processing system includes a vital sensor, a determination section and an abnormality processing section. The vital sensor detects a vital sign of a worker. The determination section determines whether or not an abnormality is present in the worker's living body based on a detected value obtained by the vital sensor. In cases where the determination section determines that an abnormality is present in the worker's living body, the abnormality processing section executes predetermined abnormality processing.

According to the above configuration, since the predetermined abnormality processing is executed in cases where an abnormality occurs in the worker's living body, appropriate processing can be executed in cases where an abnormality occurs in the worker's living body.

Preferably, the predetermined abnormality processing is processing for informing of occurrence of an abnormality.

According to the above configuration, in cases where an abnormality occurs in the worker's living body, since the occurrence of the abnormality is informed, a manufacturing defect arising from the abnormality in the worker's living body can be prevented from occurring.

Preferably, the predetermined abnormality processing is processing for stopping operation of a device used in work of the worker.

According to the above configuration, since the work device used in work of the worker stops operation in cases where an abnormality occurs in the worker's living body, a manufacturing defect arising from the abnormality in the worker's living body can be prevented from occurring.

Preferably, the abnormality processing system includes an imaging section and a memory section. The imaging section captures an image of work content performed by the worker. The memory section stores imaging data containing at least data of the image captured by the imaging section. The predetermined abnormality processing is processing for storing in the memory section the imaging data before and after a timing at which the abnormality has occurred in the worker's living body.

According to the above configuration, since the imaging data containing at least data of images before and after the timing at which the abnormality has occurred in the worker's living body is stored in the memory section, it can be specified that a manufacturing defect arises from the abnormality in the worker's living body and a recurrence prevention measure can be taken.

Preferably, the imaging section includes a first imaging section attached to the worker, and a second imaging section mounted at a predetermined place and capturing an image of the worker.

According to the above configuration, in cases where an abnormality occurs in the worker's living body, since data of an image captured from the same perspective as that of the worker and data of an image captured of the worker's action are stored in the memory section, it can be easily specified that a manufacturing defect arises from the abnormality in the worker's living body.

Preferably, the abnormality processing system includes a management computer. The management computer is connected to the vital sensor so as to communicate therewith, and includes the determination section and the determination section.

According to the above configuration, by the vital sensor and the management computer, appropriate processing can be executed in cases where an abnormality occurs in the worker's living body.

### [Effects of the Invention]

According to the invention, appropriate processing can be executed in cases where an abnormality occurs in a worker's living body.

### BRIEF DESCRIPTION OF THE DRAWINGS

FIG. 1 schematically illustrates an overall configuration of an abnormality processing system according to the present embodiment.
FIG. 2 schematically illustrates a functional configuration of the abnormality processing system according to the present embodiment.
FIG. 3 illustrates a management table stored in a management computer.
FIG. 4 is a timing chart showing an example of processing for storing imaging data in abnormal conditions when an abnormality is detected.
FIG. 5 is a flowchart for explaining abnormality processing executed by the management computer according to the present embodiment.
FIG. 6 is a flowchart for explaining abnormality processing executed by a worker camera and a surveillance camera according to the present embodiment.
FIG. 7 is a flowchart for explaining abnormality processing executed by the management computer according to a modified example.
FIG. 8 is a flowchart for explaining abnormality processing executed by the worker camera and the surveillance camera according to a modified example.

### DESCRIPTION OF THE EMBODIMENTS

Embodiments of the invention are explained in detail with reference to the drawings. Moreover, in the following drawings, the same or equivalent parts are denoted by the same reference numerals, and explanations thereof are not repeated.

### [Configuration of Abnormality Processing System]

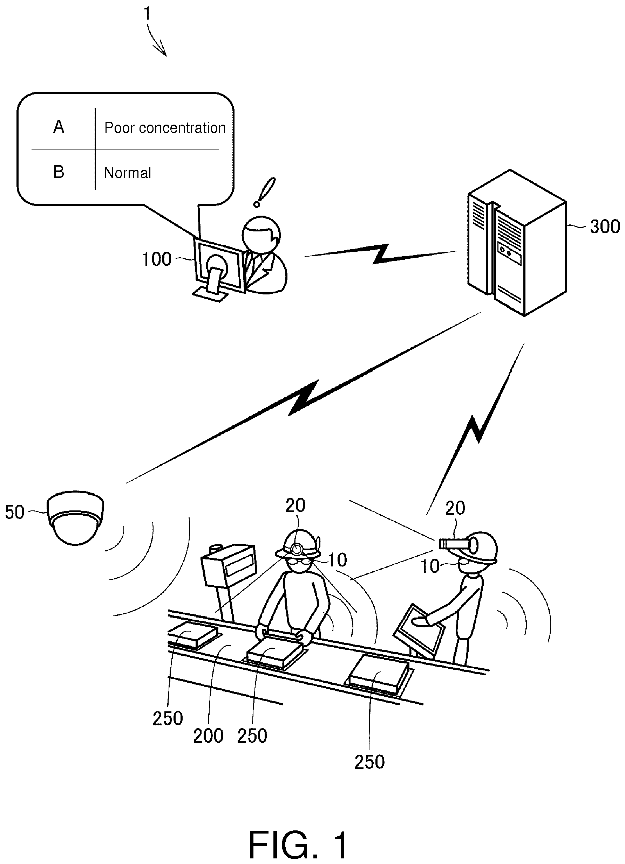

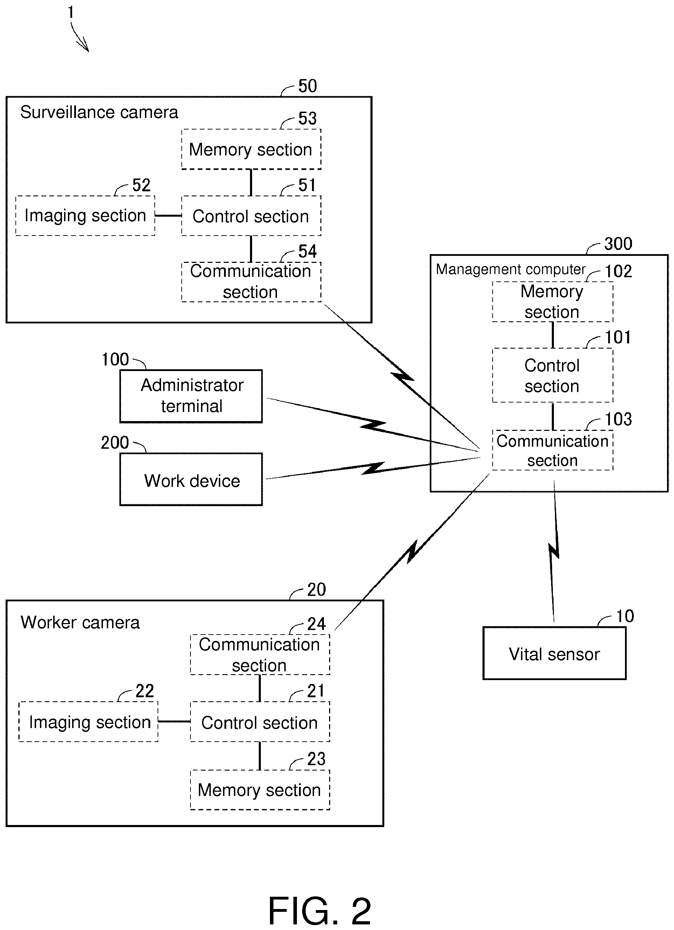

FIG. 1 schematically illustrates an overall configuration of an abnormality processing system 1 according to the present embodiment. FIG. 2 schematically illustrates a functional configuration of the abnormality processing system 1 according to the present embodiment. As shown in FIG. 1 and FIG. 2, at a work site such as a factory, a work device 200 used in work of a worker is disposed. The work device 200 is, for example, a belt conveyor, wherein semi-finished products 250 before completion which are placed on a belt are conveyed in turns by operation of the work device 200. The worker performs work of attaching a component (not illustrated) or the like to the semi-finished product 250 conveyed by the work device 200.

At such work site, in cases where an abnormality such as heating abnormality, pulse (heartbeat) abnormality, poor concentration, and sleepiness occurs in the worker's living body, a manufacturing defect is likely to occur. For example, if the worker's pulse suddenly increases, the worker may exhibit symptoms such as shortness of breath and palpitation and become careless in their work, and a manufacturing defect is likely to occur. Therefore, in the present embodiment, the abnormality processing system 1 is applicable to the work site. By the abnormality processing system 1, a manufacturing defect arising from an abnormality in the worker's living body is prevented from occurring, and recurrence of the manufacturing defect is also prevented. Hereinafter, the abnormality processing system 1 according to the present embodiment is explained.

The abnormality processing system 1 corresponds to one embodiment of the "abnormality processing system," and is a system applicable to a work site. The abnormality processing system 1 includes a vital sensor 10, a worker camera 20, a surveillance camera 50, an administrator terminal 100, a work device 200, and a management computer 300.

The vital sensor 10 is attached to a part of a worker's body and detects a vital sign being a life sign of the worker. For example, the vital sensor 10 is a glasses type vital sensor which can be worn by the worker, and detects body temperature, pulse (heartbeat), brain waves or the like as a vital sign. For example, by detecting a motion of the worker's eyelid or the like by the glasses type vital sensor 10, poor concentration and sleepiness are detected. Moreover, the vital sensor 10 may detect not only body temperature, pulse (heartbeat) and brain waves, but also other vital signs such as blood temperature and respiratory rate. In addition, the vital sensor 10 may be any vital sensor, such as a wristband type vital sensor which can be attached to the worker's wrist and a cap type vital sensor worn by the worker, as long as it can be attached to a part of the worker's body.

The worker camera 20 corresponds to one embodiment of the "imaging section" and the "first imaging section." The worker camera 20 is a video camera attached to a part of the worker's body and capturing an image of at least work content performed by the worker. For example, the worker camera 20 is attached to a helmet worn by the worker and captures an image of the work content from the same perspective as that of the worker. Imaging data obtained by image capturing by the worker camera 20 contains at least image data such as animation data and still image data. Furthermore, the imaging data obtained by the worker camera 20 in the present embodiment contains audio data in addition to the image data. Moreover, the imaging data obtained by the worker camera 20 may contain no audio data. In addition, the worker camera 20 may be attached to a place other than the helmet, such as glasses worn by the worker and the worker's wrist. In addition to capturing an image of the work content, the worker camera 20 also captures an image of a lot number of the semi-finished product 250 made by the worker. Accordingly, with the aid of the image captured by the worker camera 20, an administrator who manages workers can specify a worker who made a semi-finished product 250 in which a manufacturing defect has occurred. Furthermore, with the aid of the image captured by the worker camera 20, the administrator can also specify conditions of a work site at which the manufacturing defect has occurred, and so on.

As shown in FIG. 2, the worker camera 20 includes, as a functional configuration, a control section 21, an imaging section 22, a memory section 23 and a communication section 24. The control section 21 is a functional section corresponding to a central processing unit (CPU) controlling the imaging section 22, the memory section 23 and the communication section 24, and so on. The imaging section 22 captures an image of the work content performed by the worker based on control from the control section 21. The memory section 23 is a functional section corresponding to a random access memory (RAM) storing predetermined data. For example, the memory section 23 temporarily stores imaging data obtained by image capturing by the imaging section 22. The communication section 24 is a functional section corresponding to an antenna wirelessly communicating with the management computer 300.

The surveillance camera 50 corresponds to one embodiment of the "imaging section" and the "second imaging section." The surveillance camera 50 is a video camera mounted at a predetermined place in the factory and capturing an image of the work content performed by the worker. For example, the surveillance camera 50 is mounted on a ceiling in the factory and captures images of actions of each worker by capturing images of the whole inside of the factory. Moreover, the surveillance camera 50 may be mounted at a place other than the ceiling, such as a wall and a work table in the factory. Imaging data obtained by image capturing by the surveillance camera 50 contains at least image data such as animation data and still image data. Furthermore, the imaging data obtained by the surveillance camera 50 in the present embodiment contains audio data in addition to the image data. Moreover, the imaging data obtained by the surveillance camera 50 may contain no audio data. With the aid of the image captured by the surveillance camera 50, the administrator can also specify conditions of the work site at which the manufacturing defect has occurred, and so on.

As shown in FIG. 2, the surveillance camera 50 includes, as a functional configuration, a control section 51, an imaging section 52, a memory section 53 and a communication section 54. The control section 51 is a functional section corresponding to a CPU controlling the imaging section 52, the memory section 53 and the communication section 54, and so on. The imaging section 52 captures an image of the work content performed by the worker based on control from the control section 51. The memory section 53 is a functional section corresponding to a RAM storing predetermined data. For example, the memory section 53 temporarily stores imaging data obtained by image capturing by the imaging section 52. The communication section 54 is a functional section corresponding to an antenna wirelessly communicating with the management computer 300.

By the worker camera 20 and the surveillance camera 50 having the configurations as above, if imaging data of the work content performed by the worker is stored, it can be useful for creating a work procedure and for clarifying a cause of reduction in yield rate, and so on.

The administrator terminal 100 is a computer operated by the administrator who manages workers and the work device 200. The administrator manages a schedule of each worker and operating time of the work device 200 by using the administrator terminal 100.

The management computer 300 corresponds to one embodiment of the "management computer." The management computer 300 is a computer such as a programmable logic controller (PLC) managing each device disposed in the factory and the workers.

As shown in FIG. 2, the management computer 300 includes, as a functional configuration, a control section 101, a memory section 102 and a communication section 103. The control section 101 corresponds to one embodiment of the "determination section" and the "abnormality processing section," and is a functional section corresponding to a CPU controlling the memory section 102 and the communication section 103, and so on. The memory section 102 corresponds to one embodiment of the "memory section," and is a functional section corresponding to a RAM storing predetermined data. For example, the memory section 102 stores the imaging data received from the worker camera 20 and the surveillance camera 50 through the communication section 103. The communication section 103 is a functional section corresponding to an antenna wirelessly communicating with each of the vital sensor 10 attached to each worker, the worker camera 20 attached to each worker, the surveillance camera 50, the administrator terminal 100 and the work device 200. Moreover, the communication section 103 may wiredly communicate with each of or any of the vital sensor 10, the worker camera 20, the surveillance camera 50, the administrator terminal 100 and the work device 200.

### [One Example of Abnormality Processing]

In the abnormality processing system 1 having the configuration as above, by each vital sensor 10, a vital sign of each worker is detected. Data (hereinafter also referred to as "vital data") showing detected values obtained by each vital sensor 10 is transmitted one by one from each vital sensor 10 to the management computer 300. The management computer 300 determines whether or not an abnormality is present in the worker's living body based on a detected value which can be specified from the vital data received from each vital sensor 10 and a threshold stored in a pre-stored management table.

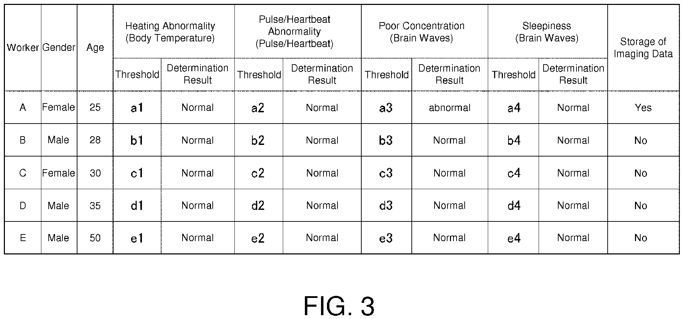

FIG. 3 illustrates a management table stored in the management computer 300. As shown in FIG. 3, the management table stores data showing each worker's name (A, B, C, D and E), data showing each worker's gender, data showing each worker's age, data relating to vital signs of each worker, and data showing presence or absence of storage of imaging data of each worker. The data relating to vital signs contains thresholds of body temperature for determining presence or absence of heating abnormality and determination results thereof, thresholds of pulse (heartbeat) for determining presence or absence of pulse (heartbeat) abnormality and determination results thereof, thresholds of brain waves for determining poor concentration and determination results thereof, and thresholds of brain waves for determining sleepiness and determination results thereof. Moreover, the thresholds are calculated based on detected values of daily vital signs of each worker. For example, a mean value calculated from the detected values of daily vital signs of each worker may be used as a threshold.

The management computer 300 compares a detected value which can be specified from the vital data received from each vital sensor 10 with a threshold of each vital sign stored in the management table shown in FIG. 3, and, if determining that the detected value exceeds the threshold, determines that an abnormality is present in the worker's living body. For example, the management computer 300 compares a detected value of brain waves which can be specified from the vital data received from the vital sensor 10 attached to A with a threshold a3 of brain waves stored in the management table shown in FIG. 3, and, if determining that the detected value of brain waves does not exceed the threshold a3, stores data (data showing normality) showing that the concentration does not decrease as a determination result in the management table. On the other hand, if determining that the detected value of brain waves exceeds the threshold a3, the management computer 300 stores data (data showing abnormality) showing that the concentration decreases as a determination result in the management table. Moreover, in the example shown in FIG. 3, the data showing that A's concentration decreases is stored in the management table.

Furthermore, if determining that an abnormality is present in the worker's living body, the management computer 300 executes predetermined abnormality processing.

As first abnormality processing, for example, if determining that an abnormality is present in the worker's living body, the management computer 300 transmits to the administrator terminal 100 an abnormality signal for informing of occurrence of the abnormality. On the other hand, when receiving the abnormality signal from the management computer 300, the administrator terminal 100 displays on a screen (not illustrated) an image showing that an abnormality has occurred. For example, when determining that A has poor concentration, the management computer 300 transmits an abnormality signal showing that A has poor concentration to the administrator terminal 100. On the other hand, as shown in FIG. 1, when receiving the abnormality signal from the management computer 300, the administrator terminal 100 displays on the screen an image showing that A has poor concentration. Accordingly, the administrator can perform appropriate processing such as suspending the work performed by A.

In this way, in cases where an abnormality occurs in the worker's living body, since the occurrence of the abnormality is informed, a manufacturing defect arising from the abnormality in the worker's living body can be prevented from occurring.

As second abnormality processing, for example, if determining that an abnormality is present in the worker's living body, the management computer 300 transmits to the work device 200 an abnormality signal for stopping operation of the work device 200. On the other hand, when receiving the abnormality signal from the management computer 300, the work device 200 stops operation. For example, when determining that A has poor concentration, the management computer 300 transmits to the administrator terminal 100 an abnormality signal indicating stoppage of operation. On the other hand, when receiving the abnormality signal from the management computer 300, the work device 200 stops operation. Moreover, when receiving the abnormality signal from the management computer 300, the work device 200 may lower its operating speed. In addition, it may be that only the work device 200 used in work of the worker who undergoes the living body abnormality stops operation or lowers its operating speed according to the abnormality signal from the management computer 300. In this case, the other work devices 200 used in work of the workers who do not undergo any living body abnormality may normally operate.

In this way, in cases where an abnormality occurs in the worker's living body, since the work device 200 stops operation or lowers its operating speed, a manufacturing defect arising from the abnormality in the worker's living body can be prevented from occurring.

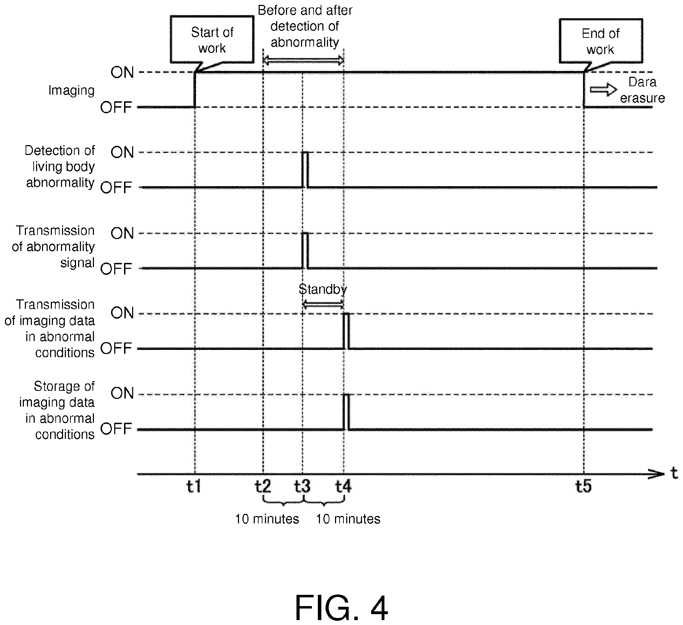

As third abnormality processing, for example, if determining that an abnormality is present in the worker's living body, the management computer 300 transmits to the worker camera 20 attached to the worker who undergoes the living body normality and the surveillance camera 50 an abnormality signal for storing images before and after a timing at which the abnormality has occurred in the worker's living body. On the other hand, when receiving the abnormality signal from the management computer 300, the worker camera 20 and the surveillance camera 50 transmit to the management computer 300 imaging data (hereinafter also referred to as "imaging data in abnormal conditions") over a predetermined time (e.g., a total of 20 minutes including 10 minutes before the timing of occurrence of the abnormality and 10 minutes after the timing of occurrence of the abnormality) over before and after the timing at which the living body abnormality has occurred. Then, the management computer 300 stores the imaging data in abnormal conditions transmitted from the worker camera 20 and the surveillance camera 50. A specific explanation regarding this third abnormality processing is given with reference to FIG. 4.

FIG. 4 is a timing chart showing an example of processing for storing imaging data in abnormal conditions when an abnormality is detected. As shown in FIG. 4, the worker camera 20 and the surveillance camera 50 continue capturing an image of the work content performed by the worker from a timing t1 of start of work until a timing t5 of end of work, and temporarily store imaging data thereof in the memory sections 23 and 53. However, if the memory sections 23 and 53 continue storing the imaging data of every work of each worker, the volume of accumulated data will become enormous. Hence, the worker camera 20 and the surveillance camera 50 erase their stored imaging data when the work ends.

At a timing t3 after start of the work, when detecting an abnormality in the worker's living body, the management computer 300 transmits an abnormality signal to the worker camera 20 attached to the worker who undergoes the living body normality and the surveillance camera 50. On the other hand, when receiving the abnormality signal from the management computer 300, the worker camera 20 and the surveillance camera 50 continue capturing an image while standing by for a predetermined time (e.g., 10 minutes) until the imaging data in abnormal conditions is complete. At a timing t4 after a predetermined time (e.g., 10 minutes) has passed since the occurrence of the abnormality in the worker's living body, the worker camera 20 and the surveillance camera 50 transmit to the management computer 300 the imaging data in abnormal conditions concerning image capturing from the timing t2 to the timing t4 over before and after the timing at which the living body abnormality has occurred. Then, at the timing t4, the management computer 300 stores the imaging data in abnormal conditions transmitted from the worker camera 20 and the surveillance camera 50. Accordingly, while the imaging data stored in the worker camera 20 and the surveillance camera 50 is erased when the work ends, the imaging data in abnormal conditions still remains after the work has ended since it is stored in the management computer 300.

In this way, since the images before and after the timing at which the abnormality has occurred in the worker's living body are stored in the management computer 300, it can be specified that a manufacturing defect arises from the abnormality in the worker's living body, and a recurrence prevention measure can be taken. Furthermore, since the imaging data stored in the worker camera 20 and the surveillance camera 50 is erased when the work ends, the accumulated data is prevented from becoming enormous in volume.

In addition, since an image captured from the same perspective as that of the worker by the worker camera 20 attached to a part of the worker's body and an image of the worker's action captured by the surveillance camera 50 mounted on the ceiling in the factory are stored in the management computer 300, it can be easily specified that a manufacturing defect arises from the abnormality in the worker's living body.

Moreover, when receiving the abnormality signal from the management computer 300, the worker camera 20 and the surveillance camera 50, while transmitting the imaging data in abnormal conditions to the management computer 300, may store the imaging data in abnormal conditions in a memory section, such as a secure digital (SD) card built in a camera, in which data will not be erased even if the power is turned off.

### [Abnormality Processing Flow]

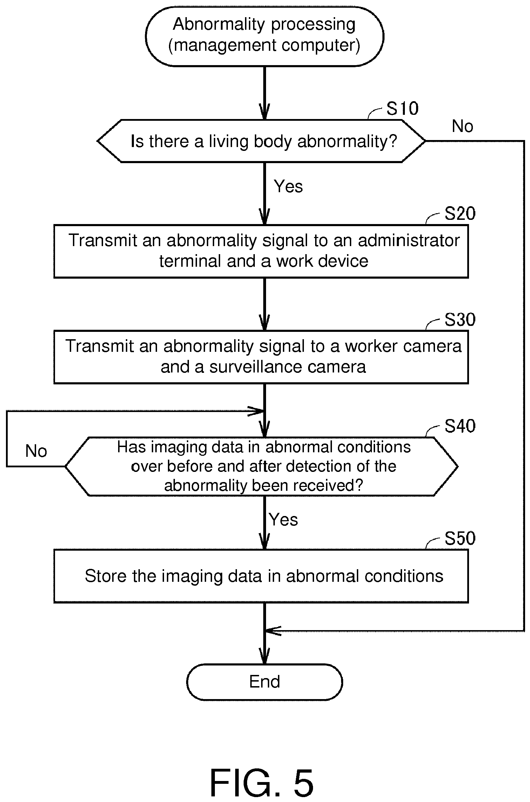

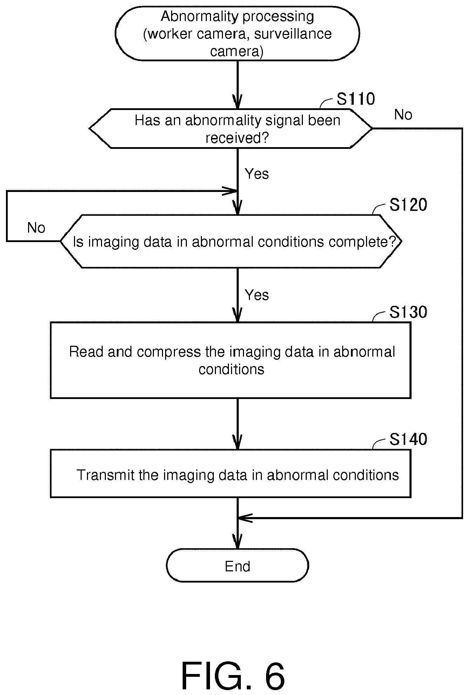

Next, the abnormality processing executed in the abnormality processing system 1 is explained with reference to FIG. 5 and FIG. 6. Moreover, the abnormality processing shown in FIG. 5 and FIG. 6 is executed at intervals of a predetermined period (e.g., 100 msec).

FIG. 5 is a flowchart for explaining the abnormality processing executed by the management computer 300 according to the present embodiment. As shown in FIG. 5, the management computer 300 determines whether or not an abnormality is present in a worker's living body based on a detected value which can be specified from vital data received from each vital sensor 10 and a threshold stored in a pre-stored management table (S10). If determining that no abnormality is present in the living body of any worker (NO in S10), the management computer 300 ends the present processing.

On the other hand, if determining that an abnormality is present in the living body of any worker (YES in S10), the management computer 300 transmits an abnormality signal to the administrator terminal 100 and the work device 200 (S20). Accordingly, an image indicating that a living body abnormality has occurred is displayed in the administrator terminal 100, and the work device 200 stops operation.

Furthermore, the management computer 300 transmits an abnormality signal to the worker camera 20 and the surveillance camera 50 (S30). After that, the management computer 300 determines whether or not imaging data in abnormal conditions has been received from the worker camera 20 and the surveillance camera 50 (S40). If the imaging data in abnormal conditions has not been received (NO in S40), the management computer 300 repeatedly executes the processing in S40 until receiving the imaging data in abnormal conditions.

On the other hand, if the imaging data in abnormal conditions has been received (YES in S40), the management computer 300 stores the received imaging data in abnormal conditions (S50) and ends the present processing.

FIG. 6 is a flowchart for explaining the abnormality processing executed by the worker camera 20 and the surveillance camera 50 according to the present embodiment. As shown in FIG. 6, the worker camera 20 and the surveillance camera 50 determine whether or not an abnormality signal has been received from the management computer 300 (S110). If the abnormality signal has not been received (NO in S110), the worker camera 20 and the surveillance camera 50 end the present processing.

On the other hand, if the abnormality signal has been received (YES in S110), the worker camera 20 and the surveillance camera 50 determine whether or not imaging data in abnormal conditions is complete (S120). That is, the worker camera 20 and the surveillance camera 50 determine whether or not 10 minutes have passed since the receipt of the abnormality signal. If the imaging data in abnormal conditions is not complete (NO in S120), the worker camera 20 and the surveillance camera 50 repeatedly execute the processing in S120 until the imaging data in abnormal conditions is complete.

On the other hand, if the imaging data in abnormal conditions is complete (YES in S120), the worker camera 20 and the surveillance camera 50 read the imaging data in abnormal conditions from the memory sections 23 and 53 and compress it (S130). After that, the worker camera 20 and the surveillance camera 50 transmit the compressed imaging data in abnormal conditions (S140) and end the present processing.

According to the abnormality processing system 1 explained as above, since predetermined abnormality processing is executed in cases where an abnormality occurs in the worker's living body, appropriate processing can be executed in cases where an abnormality occurs in the worker's living body.

### [Modified Examples]

The above has explained a main embodiment of the invention. However, the invention is not limited to the above embodiment and can be modified and applied in various ways. Hereinafter, modified examples of the above embodiment which are applicable to the invention are explained.

### (Regarding Storage of Imaging Data in Abnormal Conditions)

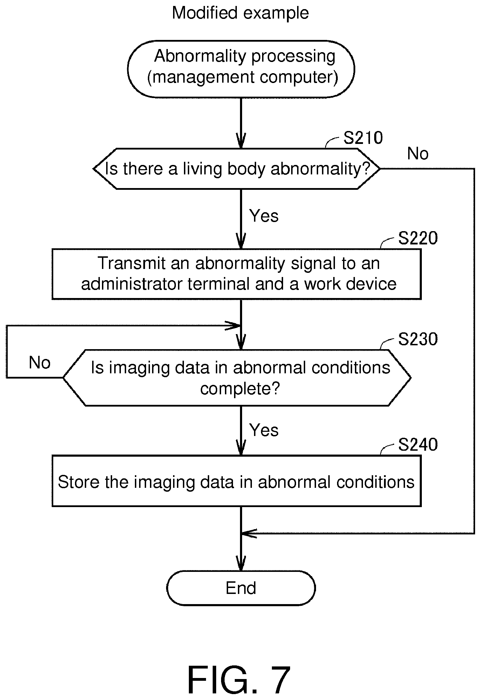

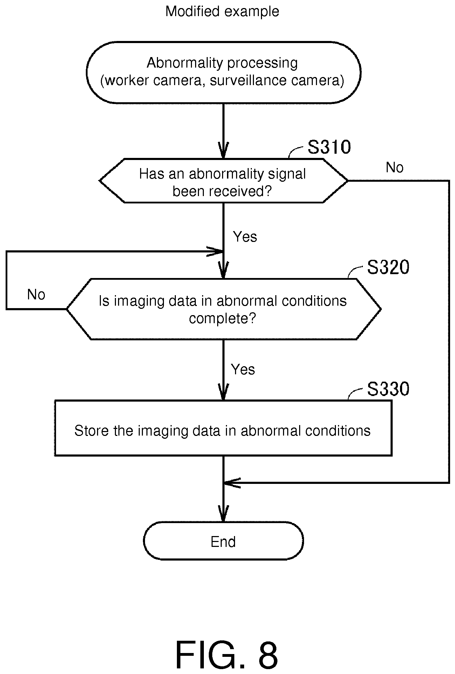

In the present embodiment, the worker camera 20 and the surveillance camera 50 continue capturing an image of the work content performed by the worker from start of work until end of work, and temporarily store the imaging data thereof. When a living body abnormality occurs, the management computer 300 stores the imaging data in abnormal conditions received from the worker camera 20 and the surveillance camera 50. However, the imaging data in abnormal conditions may be stored without being erased as in the abnormality processing shown in FIG. 7 and FIG. 8.

FIG. 7 is a flowchart for explaining the abnormality processing executed by the management computer 300 according to a modified example. In the abnormality processing shown in FIG. 7, the worker camera 20 and the surveillance camera 50 continue capturing an image of the work content performed by the worker from start of work until end of work, and transmit imaging data thereof one by one to the management computer 300. Then, the management computer 300 temporarily stores the imaging data received from the worker camera 20 and the surveillance camera 50, and, when a living body abnormality occurs, stores, as a backup, imaging data in abnormal conditions over before and after the timing at which the living body abnormality has occurred. A specific explanation is given with reference to FIG. 7.

As shown in FIG. 7, the management computer 300 determines whether or not an abnormality is present in a worker's living body (S210). If determining that no abnormality is present in the living body of any worker (NO in S210), the management computer 300 ends the present processing.

On the other hand, if determining that an abnormality is present in the living body of any worker (YES in S210), the management computer 300 transmits an abnormality signal to the administrator terminal 100 and the work device 200 (S220). Accordingly, an image indicating that a living body abnormality has occurred is displayed in the administrator terminal 100, and the work device 200 stops operation.

Furthermore, the management computer 300 determines whether or not imaging data in abnormal conditions is complete (S230). If the imaging data in abnormal conditions is not complete (NO in S230), the processing in S230 is repeatedly executed until the imaging data in abnormal conditions is complete.

On the other hand, if the imaging data in abnormal conditions is complete (YES in S230), the management computer 300 stores the imaging data in abnormal conditions as a backup in a predetermined area in the memory section 102 (S240). After that, the management computer 300 ends the present processing.

Accordingly, even if the imaging data temporarily stored in the management computer 300 is erased when the work ends, since the imaging data in abnormal conditions remains by the backup, a cause of a manufacturing defect arising from the abnormality in the worker's living body can be clarified, so as to prevent recurrence of the manufacturing defect.

FIG. 8 is a flowchart for explaining the abnormality processing executed by the worker camera 20 and the surveillance camera 50 according to a modified example. In the abnormality processing shown in FIG. 8, the worker camera 20 and the surveillance camera 50 continue capturing an image of the work content performed by the worker from start of work until end of work, and temporarily store imaging data thereof. When a living body abnormality occurs, the worker camera 20 and the surveillance camera 50 store, as a backup, imaging data in abnormal conditions over before and after the timing at which the living body abnormality has occurred. A specific explanation is given with reference to FIG. 8.

As shown in FIG. 8, the worker camera 20 and the surveillance camera 50 determine whether or not an abnormality signal has been received from the management computer 300 (S310). If the abnormality signal has not been received (NO in S310), the worker camera 20 and the surveillance camera 50 end the present processing.

On the other hand, if the abnormality signal has been received (YES in S310), the worker camera 20 and the surveillance camera 50 determine whether or not imaging data in abnormal conditions is complete (S320). If the imaging data in abnormal conditions is not complete (NO in S320), the worker camera 20 and the surveillance camera 50 repeatedly execute the processing in S320 until the imaging data in abnormal conditions is complete.

On the other hand, if the imaging data in abnormal conditions is complete (YES in S320), the worker camera 20 and the surveillance camera 50 store the imaging data in abnormal conditions as a backup in a predetermined area in each of the memory section 23 and the memory section 53 (S330). After that, the worker camera 20 and the surveillance camera 50 end the present processing.

Accordingly, even if the imaging data temporarily stored in each of the worker camera 20 and the surveillance camera 50 is erased when the work ends, since the imaging data in abnormal conditions remains by the backup, a cause of a manufacturing defect arising from the abnormality in the worker's living body can be clarified, so as to prevent recurrence of the manufacturing defect.

In the present embodiment, the imaging data in abnormal conditions obtained by image capturing is stored in each of the worker camera 20 and the surveillance camera 50. However, the imaging data in abnormal conditions obtained by image capturing may be stored in only either of the worker camera 20 and the surveillance camera 50.

In the present embodiment, the imaging data in abnormal conditions is imaging data over a predetermined time over before and after the timing at which an abnormality has occurred in the worker's living body. However, the imaging data in abnormal conditions may be imaging data from the timing at which an abnormality has occurred in the worker's living body to when a predetermined time (e.g., 10 minutes) has passed. In addition, the imaging data in abnormal conditions may be imaging data from a predetermined time (e.g., 10 minutes) before the timing at which an abnormality has occurred in the worker's living body until when the worker's living body returns to normal. Moreover, imaging time in the imaging data in abnormal conditions may be set in advance by the administrator or the like. For example, in cases where a coating work or the like is performed, in view of coating time, the administrator may set the imaging time in the imaging data in abnormal conditions slightly longer than that in normal work.

### (Regarding Camera)

In the present embodiment, images of the work content performed by the worker are captured using two cameras, including the worker camera 20 attached to the worker and the surveillance camera 50 mounted at a predetermined place in the factory. However, images of the work content performed by the worker may be captured by only either of the worker camera 20 and the surveillance camera 50. Alternatively, images of the work content performed by the worker may be captured using another camera different from the worker camera 20 and the surveillance camera 50.

In the present embodiment, the worker camera 20 and the surveillance camera 50 continue capturing an image of the work content performed by the worker from start of work until end of work, and erase their stored imaging data when the work ends. However, the worker camera 20 and the surveillance camera 50 may continue to store the imaging data for a certain period (e.g., 24 hours, 1 week, etc.) without erasure even after the work has ended. In addition, during work, the worker camera 20 and the surveillance camera 50 may erase the imaging data after capturing an image for a certain period (e.g., 3 hours, etc.).

In the present embodiment, the worker camera 20 and the vital sensor 10 are separate from each other. However, a device having functions of both the worker camera and the vital sensor may be attached to the worker. For example, by a camera attached to the glasses type vital sensor 10, poor concentration and sleepiness are detected by detecting a motion of the worker's eyelid, and an image of the work content performed by the worker may also be captured.

### (Regarding Determination of Living Body Abnormality)

In the present embodiment, a living body abnormality is determined using a threshold calculated based on a detected value of a daily vital sign of each worker. However, a detected value of a vital sign of each worker at the start of work may also be used as a threshold. In addition, the threshold is not necessarily set per worker, and mean values calculated based on gender and age and so on may also be used as thresholds.

In the present embodiment, the management computer 300 determines whether or not an abnormality is present in the worker's living body based on a vital sign received from the vital sensor 10. However, the vital sensor 10 may determine whether or not an abnormality is present in the worker's living body based on a vital sign detected by the vital sensor 10 itself. In this case, if determining that an abnormality is present in the worker's living body, the vital sensor 10 may transmit a signal showing that an abnormality is present in the worker's living body to the management computer 300. Alternatively, if determining that an abnormality is present in a worker's living body, the vital sensor 10 transmits an abnormality signal to the administrator terminal 100, the work device 200, the worker camera 20 and the surveillance camera 50.

### (Regarding Configuration of Abnormality Processing System)

In the present embodiment, the abnormality processing system 1 has the configuration shown in FIG. 1 and FIG. 2. However, the abnormality processing system 1 is not limited to such configuration. For example, the functions owned by the vital sensor 10 and the functions owned by the worker camera 20 may be owned by one device.

In addition, in the abnormality processing system 1, in cases where an abnormality occurs in the worker's living body, at least any one of the first abnormality processing for displaying an image indicating that a living body abnormality has occurred in the administrator terminal 100, the second abnormality processing for stopping operation of the work device 200 and the third abnormality processing for storing the imaging data in abnormal conditions may be performed.

Moreover, the embodiments disclosed herein are examples in all aspects and should not be interpreted as limitations. The scope of the invention is defined by claims instead of the above descriptions, and it is intended to include all modifications within the scope of the claims and the equivalents thereof.

## Claims

1. An abnormality processing system (1), comprising:
a vital sensor (10), detecting a vital sign of a worker;
a determination section, determining whether or not an abnormality is present in the worker's living body based on a detected value obtained by the vital sensor (10); and
an abnormality processing section, executing predetermined abnormality processing in cases where the determination section determines that the abnormality is present in the worker's living body.

2. The abnormality processing system (1) according to claim 1, wherein the predetermined abnormality processing is processing for informing of occurrence of the abnormality.

3. The abnormality processing system (1) according to claim 1 or 2, wherein the predetermined abnormality processing is processing for stopping operation of a device used in work of the worker.

4. The abnormality processing system (1) according to any one of claims 1 to 3, comprising:
an imaging section (22, 52), capturing an image of work content performed by the worker; and
a memory section (23, 53, 102), storing imaging data containing at least data of the image captured by the imaging section (22, 52), wherein
the predetermined abnormality processing is processing for storing the imaging data before and after a timing at which the abnormality has occurred in the worker's living body in the memory section (23, 53, 102).

5. The abnormality processing system (1) according to claim 4, wherein the imaging section (22, 52) comprises a first imaging section (20) attached to the worker, and a second imaging section (50) mounted at a predetermined place and capturing an image of the worker.

6. The abnormality processing system (1) according to any one of claims 1 to 5, comprising a management computer (300), the management computer (300) being connected to the vital sensor (10) so as to communicate therewith and comprising the determination section and the abnormality processing section.
